# EUROPEAN PATENT SPECIFICATION

(11) **EP 4 289 454 B1**
(45) Date of publication and mention of the grant of the patent: **02.09.2026**
(21) Application number: 22177576.0
(22) Date of filing: 07.06.2022
(51) Int. Cl.: A61M 5/315, A61M 5/20, A61M 5/32

(54) **REUSABLE DIGITAL MODULE FOR AUTOINJECTOR**
WIEDERVERWENDBARES DIGITALES MODUL FÜR AUTOINJEKTOR
MODULE NUMÉRIQUE RÉUTILISABLE POUR INJECTEUR AUTOMATIQUE

(43) Date of publication of application: 13.12.2023
(73) Proprietor: Becton, Dickinson and Company, Franklin Lakes, NJ 07417-1880 (US)
(72) Inventor: ALBENGE, Olivier, 38130 Echirolles (FR); CHARVIN, Matthieu, 38000 Grenoble (FR); NANAYAKKARA, Prasannah Wellalage, London N1 1EA (GB); LESTER, Robert, London SW11 5EQ (GB); PATEL, Dhruv, Ilkeston DE7 8QT (GB); KING, Brady, Tunbridge Wells TN2 4RE (GB); MASON, Jonathan Edward, Maidenhead 5L6 7JF (GB)
(74) Representative: Germain Maureau

(56) References cited:
- WO-A1-2020/261270
- US-A1- 2019 282 761
- US-A1- 2020 327 974

## Description

### BACKGROUND OF THE INVENTION

### Field of the Invention

The present disclosure relates generally to a drug delivery device and, more specifically, to an auto-injector.

### Description of Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. Many of these devices, such as auto-injectors, are designed so that the reservoir, such as a pre-filled syringe, is assembled into the device during assembly of the device. In addition to automatically deploying the needle-injection mechanism, many drug delivery devices also automatically shield the needle after use of the device to prevent any unintended contact with the needle.

Such drug delivery devices have traditionally lacked features that would allow a healthcare provider to automatically record or capture data showing appropriate administration of medicaments. Ensuring timely, appropriate administration of some medicaments can be critical, particularly, for example, in the instance of the evaluation of new drugs in clinical trials, where accurate information is essential. It can also be advantageous to include such functionality without altering the exterior appearance of drug delivery devices, adding additional steps for the user, and without adding additional complexity to the interior of the device.

While some devices provide the ability to communicate certain types of information, for example as described in International Patent Application Publication Nos. WO 2020/261270, WO 2016/087512, WO 2017/070391, WO 2018/111969, WO 2018/213837, and WO 2021/094797, and in U.S. Patent Application Publication Nos. 2019/282761, 2020/327974, 2019/0083708, 2019/0321555, and 2019/0344019, a need exists in the art for a cost-effective, reusable device that accurately detects drug delivery, and can communicate this drug delivery information to appropriate stakeholders in a timely fashion.

### SUMMARY OF THE INVENTION

Provided herein is a digital module for an autoinjector, including a housing having a distal end and a proximal end and configured to releasably mate with an autoinjector, and a first circuit board received within the housing, the circuit board having a proximal end and a distal end and including at least one switch, at least one processor in communication with the at least one switch, at least one communication interface, and at least one power source, where the digital module is as further defined in claim 1.

The at least one circuit board may be a printed circuit board.

The housing defines a channel configured to receive a plunger body of the autoinjector. The digital module also includes a second circuit board including at least one switch, at least one processor in communication with the at least one switch, at least one communication interface, and at least one power source. The first circuit board and the second circuit board may be received within the housing on opposite sides of the channel.

Also provided herein is an autoinjector having a housing having a proximal end and a distal end, a syringe including a barrel, a stopper, and a needle arranged at a distal end of the syringe, at least a portion of the syringe positioned within the housing, a drive assembly including a drive member and a plunger body, the drive assembly configured to move the stopper within the barrel upon actuation of the drive assembly, at least a portion of the drive assembly positioned within the housing, a needle cover having a pre-use position where the needle is positioned within the needle cover, an actuation position where the needle cover has been shifted proximally, thereby allowing the drive assembly to be actuated, and a post-use position where the needle is positioned within the needle cover, and a digital module, including a housing having a distal end and a proximal end and configured to releasably mate with the autoinjector housing, and a first circuit board received within the housing, the circuit board having a proximal end and a distal end and including at least one switch, at least one processor in communication with the at least one switch, at least one communication interface, and at least one power source, wherein the at least one switch is activated by the plunger body as the plunger body moves distally during actuation of the drive assembly, and wherein activation of the at least one switch indicates beginning of dose delivery and/or end of dose delivery.

In certain configurations, the at least one switch includes at least one proximal sensor and at least one distal sensor, and wherein activation of the proximal sensor indicates beginning of dose delivery and activation of the distal sensor indicates end of dose delivery. The plunger body may include a rack having a plurality of teeth, and wherein the at least one switch detects movement of the plunger body based on passage of one or more teeth. Optionally, the plunger body further includes at least a first flange configured to actuate the at least one switch, and wherein actuation of the at least one switch by the flange indicates beginning of dose delivery.

The plunger body may include at least a second flange configured to actuate the at least one switch, and wherein actuation of the at least one switch by the flange indicates end of dose delivery. The autoinjector also includes a second circuit board including at least one switch, at least one processor in communication with the at least one switch, at least one communication interface, and at least one power source. The first circuit board and the second circuit board may be received within the housing on opposite sides of the channel.

### BRIEF DESCRIPTION OF THE DRAWINGS

**FIG. 1** is a side view of a drug delivery device with a digital module according to non-limiting embodiments described herein;
**FIG. 2** is a cross-sectional view of a drug delivery device with a digital module according to non-limiting embodiments described herein;
**FIG. 3** is a perspective view of a digital module according to non-limiting embodiments described herein;
**FIG. 4** is a perspective view of a plunger body and a digital module according to non-claimed embodiments described herein;
**FIG. 5** is a perspective view of a plunger body and a digital module according to non-claimed embodiments described herein;
**FIG. 6** is a top view of a plunger body and a digital module according to non-claimed embodiments described herein;
**FIGS. 7A** and **7B** are partial top views of a drug delivery device with a digital module according to non-claimed embodiments described herein;
**FIG. 8** is a perspective view of a plunger body and a digital module according to non-limiting embodiments described herein;
**FIG. 9** is a perspective view of a plunger body and a digital module according to non-limiting embodiments described herein;
**FIG. 10** is a top view of a plunger body and a digital module according to non-limiting embodiments described herein;
**FIGS. 11A** and **11B** are partial top views of a drug delivery device with a digital module according to non-limiting embodiments described herein;
**FIG. 12** is a schematic of operation of a digital module according to non-limiting embodiments described herein;
**FIG. 13A** is a schematic of operation of a digital module according to non-claimed embodiments described herein. **FIGS. 13B-13D** are schematics of operation of a digital module according to non-limiting embodiments described herein;
**FIG. 14** is a partial side view of a digital module according to non-limiting embodiments described herein;
**FIG. 15** is a diagram of a non-limiting embodiment or aspect of an environment in which systems, devices, and/or methods described herein may be implemented; and
**FIG. 16** is a diagram of a non-limiting embodiment or aspect of components of one or more devices of **FIG. 15****.**

### DESCRIPTION OF THE INVENTION

The following description is provided to enable those skilled in the art to make and use the described embodiments contemplated for carrying out the invention. Various modifications, variations, and alternatives, however, will remain readily apparent to those skilled in the art. Any and all such modifications, variations, equivalents, and alternatives are intended to fall within the scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

As used herein, the terms "communication" and "communicate" refer to the receipt or transfer of one or more signals, messages, commands, or other type of data. For one unit (e.g., any device, system, or component thereof) to be in communication with another unit means that the one unit is able to directly or indirectly receive data from and/or transmit data to the other unit. This may refer to a direct or indirect connection that is wired and/or wireless in nature. Additionally, two units may be in communication with each other even though the data transmitted may be modified, processed, relayed, and/or routed between the first and second unit. For example, a first unit may be in communication with a second unit even though the first unit passively receives data and does not actively transmit data to the second unit. As another example, a first unit may be in communication with a second unit if an intermediary unit processes data from one unit and transmits processed data to the second unit. It will be appreciated that numerous other arrangements are possible. Any known electronic communication protocols and/or algorithms can be used such as, for example, TCP/IP (including HTTP and other protocols), WLAN (including 902.11a/b/g/n and other radio frequency-based protocols and methods), analog transmissions, Global System for Mobile Communications (GSM), 3G/4G/LTE, BLUETOOTH, NFC, RFID, ZigBee, EnOcean, TransferJet, Wireless USB, Narrowband IoT (NBIoT), low-power, wide area networking protocol (LoraWan), ultra-wideband (UWB), and the like known to those of skill in the art. In some non-limiting embodiments, a message may refer to a network packet (e.g., a data packet and/or the like) that includes data.

As used herein, the term "computing device" may refer to one or more electronic devices configured to process data. A computing device may, in some examples, include the necessary components to receive, process, and output data, such as a processor, a display, a memory, an input device, a network interface, and/or the like. A computing device may be a mobile device. As an example, a mobile device may include a cellular phone (e.g., a smartphone or standard cellular phone), a portable computer, a wearable device (e.g., watches, glasses, lenses, clothing, and/or the like), a personal digital assistant (PDA), and/or other like devices. A computing device may also be a desktop computer or other form of non-mobile computer, including a server, as well as distributed computing paradigms, such as Edge computing.

Provided herein are a digital module for a drug delivery device, a drug delivery device including such a module, and systems and methods including use of such a device, for monitoring delivery of a medicament with the drug delivery device. The devices, systems, and methods described herein improve upon traditional devices and methods of determining administration of medicaments, by enabling timely and accurate monitoring of drug delivery by relevant stakeholders in a cost-effective manner, improving, for example, clinical trials for newly-developed medicaments with more robust data management solutions.

International Patent Application Publication Nos. WO 2020/173991, WO 2020/173992, WO 2020/173993, WO 2020/173994, and WO 2020/173995 are to be referred to.

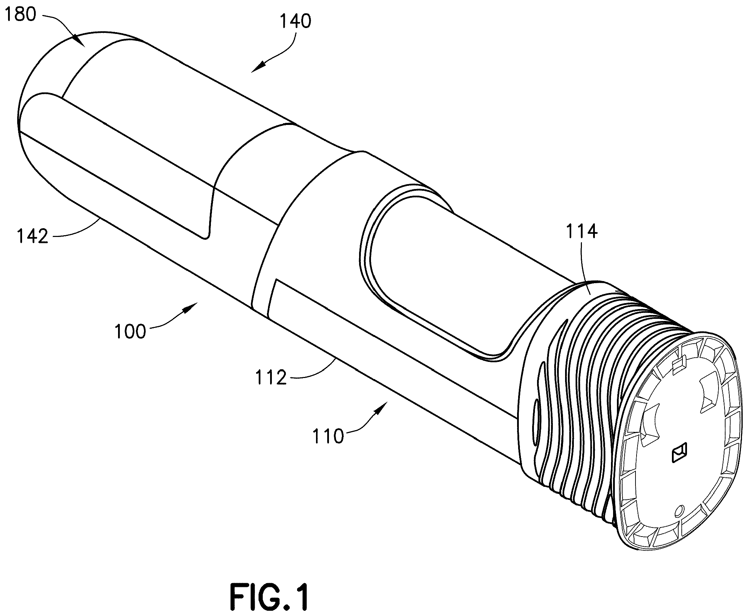

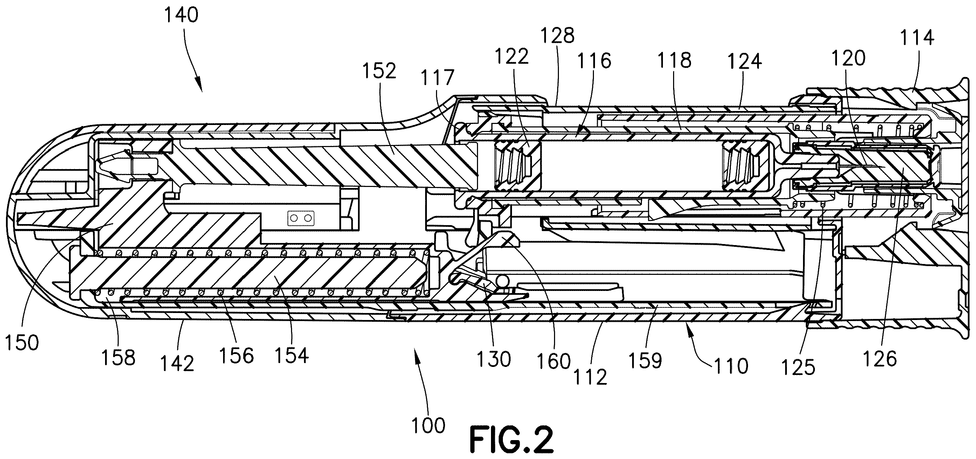

Turning to FIGS. 1 and 2, shown is a drug delivery device 100. Drug delivery device 100 may include a first subassembly 110, a second subassembly 140, and a syringe 116. The first subassembly 110 may include lower housing shell 112, a cap 114, a syringe holder 118, a needle cover 124, and a cassette body 128. The second subassembly 140 may include a drive assembly including a plunger body 150, the plunger body 150 including a plunger rod 152 and a spring guide member 154 including a drive member 156 and a drive opening 158. Second subassembly 140 may further include a motor body 159, a lever actuation member 130, and an upper housing shell 142. The syringe 116 may be received by the syringe holder 118 and includes a barrel 117, a stopper 122, a needle 120, and a rigid needle shield (RNS) 126. Although an RNS is utilized, other suitable needle shield arrangements may be utilized. The lower housing shell 112, the cassette body 128, and the upper housing shell 142 generally form a housing for receiving the various components of the drug delivery device 100, although other suitable housing arrangements may be utilized. The first subassembly 110 and the second subassembly 140 may be secured to each other during assembly by a locking clip, although other suitable arrangements may be utilized. The drug delivery device 100 may be an auto-injector, although the features described herein may be incorporated into other suitable drug delivery devices.

Drug delivery device 100 is configured to automatically deliver a dose of medicament from the syringe 116 to a patient upon actuation of the device 100. More specifically, upon actuation of the drug delivery device 100, the drive assembly is configured to engage the stopper 122 of the syringe 116, displace the syringe 116 such that the needle 120 pierces the skin of the patient, and displace the stopper 122 within the barrel 117 of the syringe 116 to deliver the medicament within the barrel 117. The drug delivery device 100 includes a storage position, a pre-use position, an actuation position, an injection position, and a post-use position, as described in International Patent Application Publication Nos. WO 2020/173991, WO 2020/173992, WO 2020/173993, WO 2020/173994, and WO 2020/173995. Needle cover 124 is configured to shield the needle 120 of the syringe 116 from the patient when the device 100 is in the pre-use and the post-use positions. In particular, the needle cover 124 is moveable between a pre-use position, an actuation position, and a post-use positon, with a spring 125 biasing the needle cover 124 towards the pre-use position and the post-use position. The spring 125 may be positioned between the needle cover 124 and the syringe holder 118, although other suitable arrangements may be utilized. The lever actuation member 130 is moveable between a locked position where movement of the drive assembly is prevented and a released position where movement of the drive assembly is allowed. More specifically, the lever actuation member 130 is rotatable about a rotation axis between the locked position and the released position. When the lever actuation member 130 is in the locked position, the lever actuation member 130 is engaged with the motor body 159 and the drive assembly to prevent movement of the drive assembly. When the lever actuation member 130 is in the released position, which may be achieved by pressing the needle cover 124 onto the patient's skin at the site of injection, shifting the needle cover proximally into the lower housing shell 112, the lever actuation member 130 is disengaged from the motor body 159 thereby allowing movement of the drive assembly toward the syringe 116. The rotation axis of the lever actuation member 130 extends perpendicular to a longitudinal axis of the device 100, although other suitable arrangements may be utilized.

Referring again to FIGS. 1 and 2, the drive assembly includes a plunger body 150 having a plunger rod portion 152 and a drive member 156. The drive member 156 may be a compression spring received within a drive opening 158 defined by the plunger body 150, although other suitable drive members may be utilized, including, but not limited to, compressed gas, an electric motor, hydraulic pressure, other types of springs, etc. The drive member 156 engages the plunger body 150 and the motor body 159 and biases the plunger body 150 in a direction extending from the second subassembly 140 toward the first subassembly 110. The plunger body 150 defines a lever opening that receives the lever actuation member 130 and defines the rotation axis of the lever actuation member 130. The lever actuation member 130 prevents movement of the plunger body 150 when the lever actuation member 130 is in the locked position through engagement of the lever actuation member 130 with the motor body 159. Upon rotation of the lever actuation member 130 from the locked position to the released position, the lever actuation member 130 is disengaged from the motor body 159 thereby allowing the drive member 156 to move the plunger body 150 and the plunger rod 152 toward the first subassembly 110. The plunger rod 152 and the drive member 156 are spaced from and parallel to each other and extend in a longitudinal direction of the device 100.

The drive assembly further includes a spring guide member 154 secured to the upper housing shell 142 and received within the drive opening 158 of the plunger body 150. The drive member 156 is received by the spring guide member 154 such that the drive member 156 is positioned between the plunger body 150 and the spring guide member 154. The drive assembly may also include a plunger rod cover that receives the plunger rod 152 of the plunger body 150. The plunger rod cover may be configured to guide insertion of the plunger rod 152 into the barrel 117 of the syringe 116 and engage the stopper 122 to dispense the medicament from the barrel 117 of the syringe 116. The plunger rod cover and the plunger rod 152 may be formed integrally or formed as separate components.

The plunger body 150 of the drive assembly may also include an audio indicator member 160 configured to provide an audible indication to a user when the device 100 transitions to the post-use position. The audio indicator member 160 may be configured to engage one or more ribs of the cassette body 128 when the device 100 is in the injection position thereby deflecting the audio indicator member 160. When the drug delivery device 100 transitions from the injection position to the post-use position, the audio indicator member 160 may disengage from the rib(s) of the cassette body 128 and contacts the lower housing shell 112 to provide an audible click, although the audio indicator member 160 could also contact other suitable portions of the device 100 to provide the audible indicator.

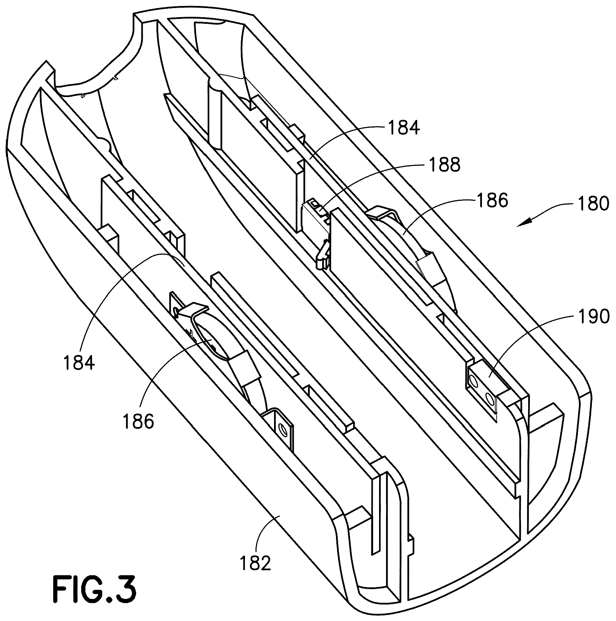

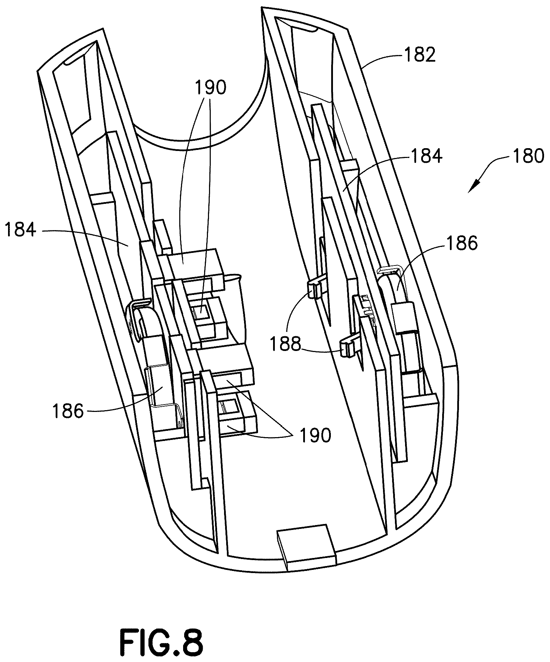

With continuing reference to FIG. 1, drug delivery device 100 further includes a digital module 180. Digital module 180 is reversibly attachable to the housing of drug delivery device 100, for example to upper housing shell 142. With reference to FIGS. 3 and 8, digital module includes a housing 182, and digital module 180 is reversibly attachable to drug delivery device 100, for example through a hinged connection between upper housing shell 142 and housing 182, a slide connection, a snap connection, a friction fit, and/or other interactions known to those of skill in the art.

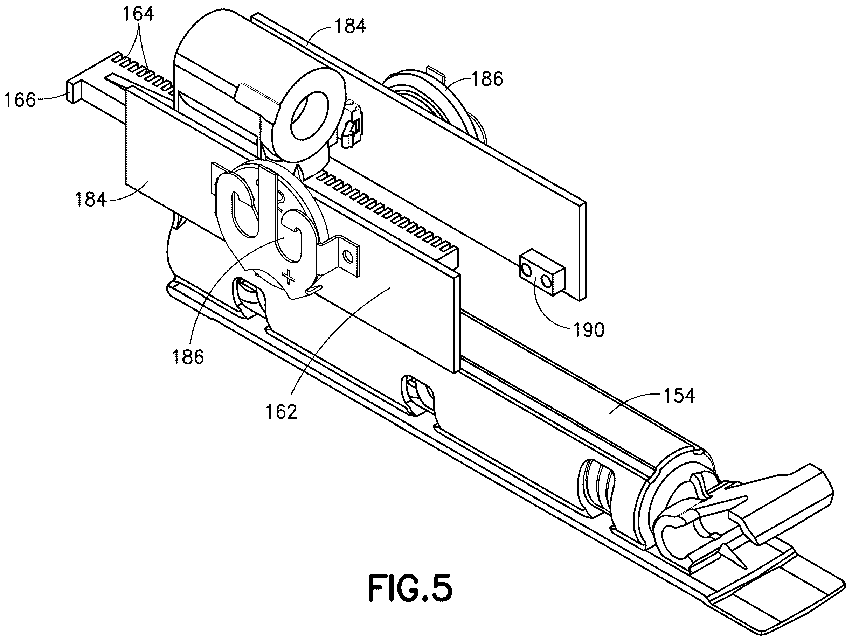

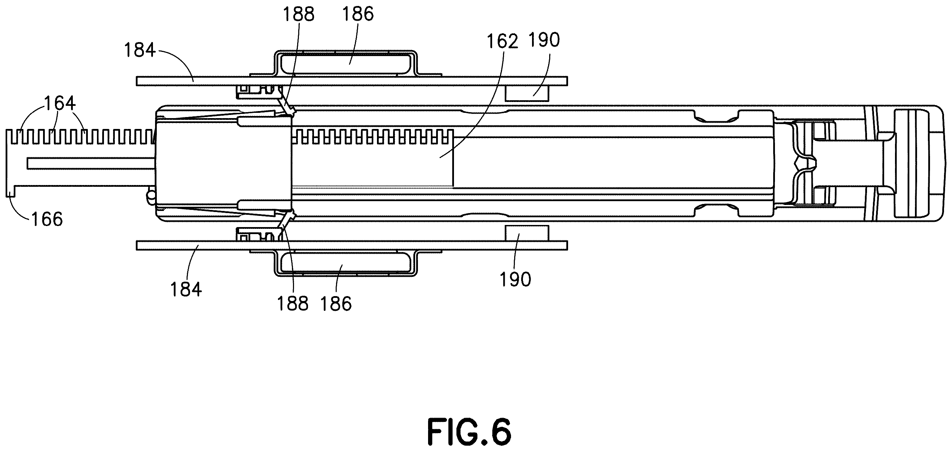

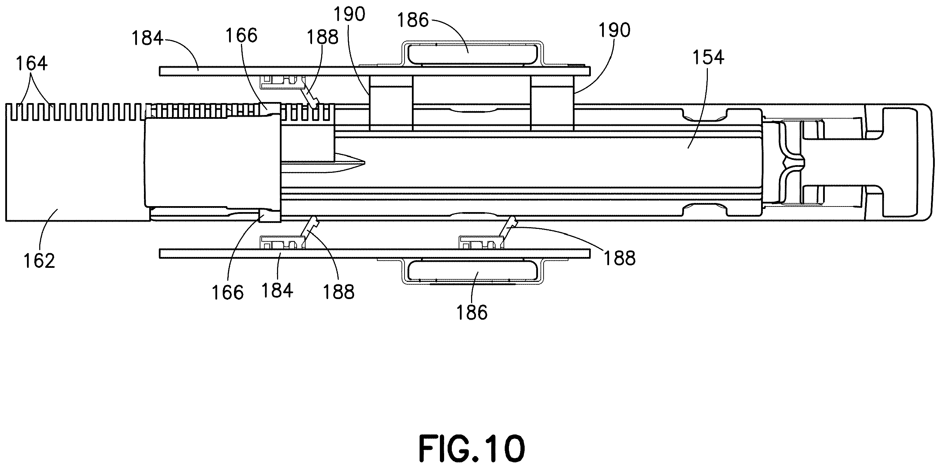

Digital module 180 further includes a first and a second circuit boards, comprising one or more power sources, one or more processors, one or more communication interfaces, and one or more switches. FIGS. 3-13D show various non-limiting embodiments of the arrangement of components of digital module 180. In non-limiting embodiments, circuit board(s) can be printed circuit boards, flexible substrates for printed electronics, and the like as are known in the art. In non-limiting embodiments, more than one circuit board is included, and the circuit boards are connected, optionally in electronic communication with each other. In non-limiting embodiments, the switches can be, independently, optical sensors, ultrasonic sensors, capacitive sensors, force resistive sensors, electrical switches, electromagnetic switches, and/or mechanical switches, and can be in communication with one or more processors on the circuit board(s). In non-limiting embodiments, digital module 180 is reusable, and suitable rechargeable or replaceable power sources can be used.

Referring to FIGS. 3-7B, shown is a non-claimed embodiment of digital module 180, including housing 182, circuit board 184, power source 186, mechanical switch(es) 188, and optical sensor(s) (e.g., optical switches) 190. Suitable mechanical switches are known to those of skill in the art, and can include the ESE13V05 from Panasonic (Kadoma, Osaka, Japan), and the HDT0001 switch produced by C&K (Waltham, MA). Suitable optical sensors are known to those of skill in the art, and can include reflective encoders, such as the AEDR-9820 from Broadcom (San Jose, CA), slotted light gate sensors such as the TCUT1300X01 from Vishay (Malvern, PA), and reflective optical sensors, such as the OPB9000 from TT Electronics (Woking, United Kingdom). In non-limiting embodiments, a reflective code strip may be applied to plunger body 150, and may thus be sensed by optical switch 190 as plunger body 150 moves distally during dose delivery. Suitable reflective code strips are known to those of skill in the art, and can include those from Photo Solutions (Wilsonville, OR), Meltec (Nagareyama, Nagareyama City, Chiba, Japan), GM Nameplate (Seattle, WA), and pwb (Eisenach, Germany). In non-limiting embodiments (discussed below), a rack may be applied to, or co-molded as part of, plunger body 150, and the rack may include one or more teeth and/or flanges to actuate switches 188 and/or 190.

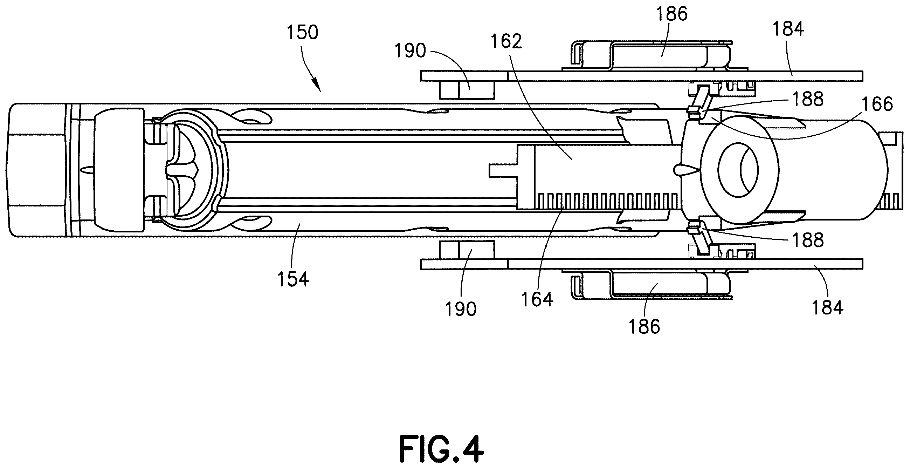

In the illustrated embodiment of FIGS. 3-7B, each circuit board 184 includes two switches, an optical sensor 190 arranged distally of a mechanical switch 188. As noted above, any suitable number, combination, and arrangement of switches may be used. In non-limiting embodiments, at least one switch is positioned such that it is actuated about 2.2 mm, about 2 mm, about 1.5 mm, about 1.1 mm, or about 1 mm, all values and subranges therebetween inclusive, before the stopper 122 reaches its distal-most position (e.g., end of dose delivery). In the non-limiting embodiment of FIGS. 3-7B, circuit boards 184 are arranged in housing 182 in a manner that provides a channel between the two circuit boards, with one or more of the switches 188, 190 arranged facing the channel. As is shown in FIGS. 4-7B, this allows for components of the drive assembly of drug delivery device 100 to be received in the channel, and to interact with the switches.

Turning now to FIGS. 4-6, shown are plunger body 150 and digital module 180 (with housing 182 not shown). In embodiments, plunger body 150 includes a rack 162. In non-limiting embodiments, rack 162 may include one or more teeth 164. Teeth 164 may be arranged on rack 162 in any suitable configuration. In non-limiting embodiments, teeth 164 are arranged at an interval of about 0.1 mm, 0.2 mm, 0.3 mm, 0.4 mm, 0.5 mm, 0.6 mm, 0.7 mm, 0.8 mm, 0.9 mm, or 1.0 mm, all values and subranges therebetween inclusive. Those of skill will appreciate that the number of optical switches 190, and the interval between teeth 164, may be modified to increase resolution or robustness of the switches and the information they transmit. Rack 162 may be arranged on plunger body 150 in a manner that allows for one or more teeth 164 to interact with one or more optical sensors 190. In non-limiting embodiments, rack 162 and/or plunger body 150 may also include one or more flanges 166. Plunger body 150 and/or rack 162 may be arranged in a manner that allows for one or more flanges 166 to interact with one or more mechanical switches 188.

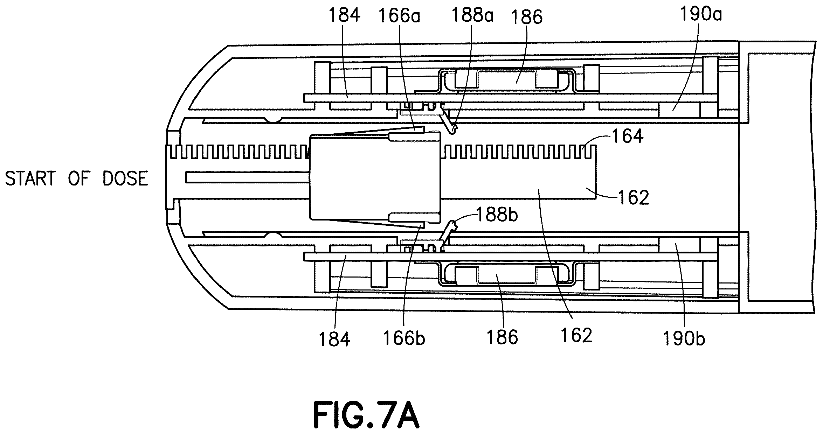

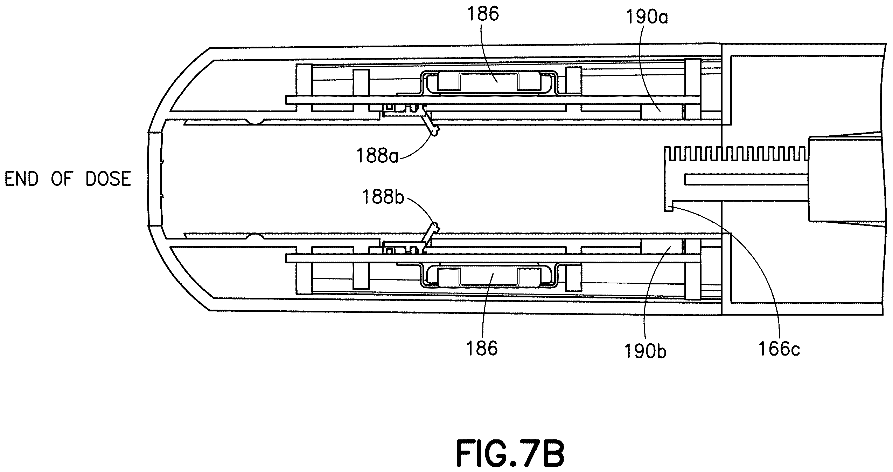

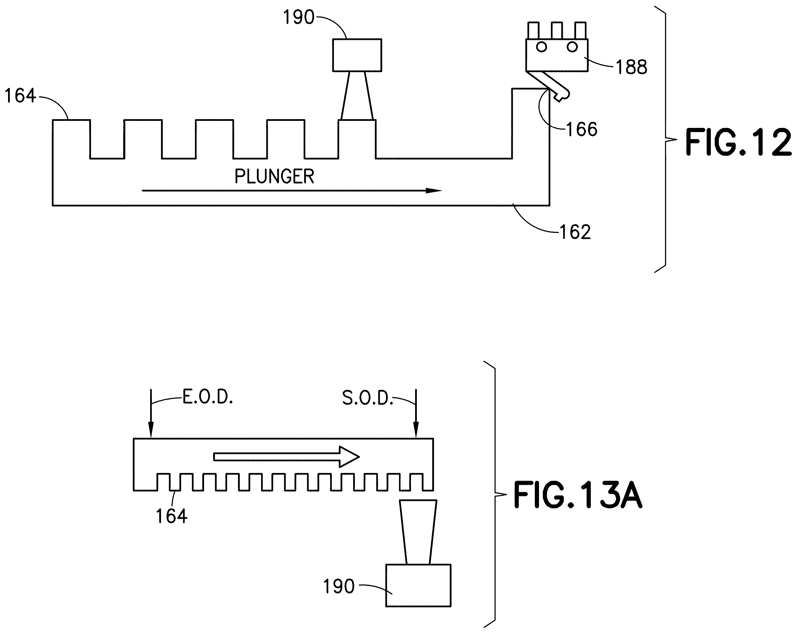

Referring now to FIGS. 7A-7B, shown are images of drug delivery device 100 in a pre-use (FIG. 7A) and post-use (FIG. 7B) position, with respect to the digital module 180. As shown in FIG. 7A, prior to actuation of the drive assembly, rack 162 is in a proximal position, and mechanical switches 188a, b have not been actuated. After drive assembly is actuated, plunger body 150, with rack 162, begins moving distally. One or more flanges 166a, b, c contact one or more mechanical switches 188a, b, causing the digital module 180 to awaken and begin recording data (as will be described in greater detail below). In non-limiting embodiments, digital module 180 may include one or more gyroscopes or other positioning sensors, such that the digital module 180 awakens when the device is picked up by a user. As will be described in greater detail below (with reference to FIGS. 12-15), switches may be arranged, and processors included on circuit boards 184 may be programmed, to determine just the beginning and end of drug delivery, and/or may be arranged and programmed to continuously record data throughout drug delivery. In non-limiting embodiments, a digital module 180 may include one or both arrangements. For example in non-limiting embodiments, digital module 180 may include both arrangements, as shown in FIGS. 7A-7B. In the illustrated embodiment of FIGS. 7A-7B, one or more teeth 164 on rack 162 interact with one or more optical sensors 190a, b, in the illustrated embodiment reflective optical sensors 190, and data may thus be continuously recorded during the drug delivery procedure. In the non-limiting embodiment of FIGS. 7A-7B, optical sensor 190b senses passage of flange 166c, indicating the end of drug delivery.

Turning to FIGS. 8-11B, shown is a non-limiting embodiment of a digital module 180, which includes housing 182 (shown in FIG. 8 only), circuit board 184, power source 186, mechanical switch(es) 188, and optical sensor(s) 190. In the non-limiting embodiment of FIGS. 8-11B, circuit boards 184 are arranged in housing 182 in a manner that provides a channel between the two circuit boards 184, with one or more of the switches 188, 190 arranged facing the channel. As is shown in FIGS. 8-11B, this allows for components of the drive assembly of drug delivery device 100 to be received in the channel, and to interact with the switches.

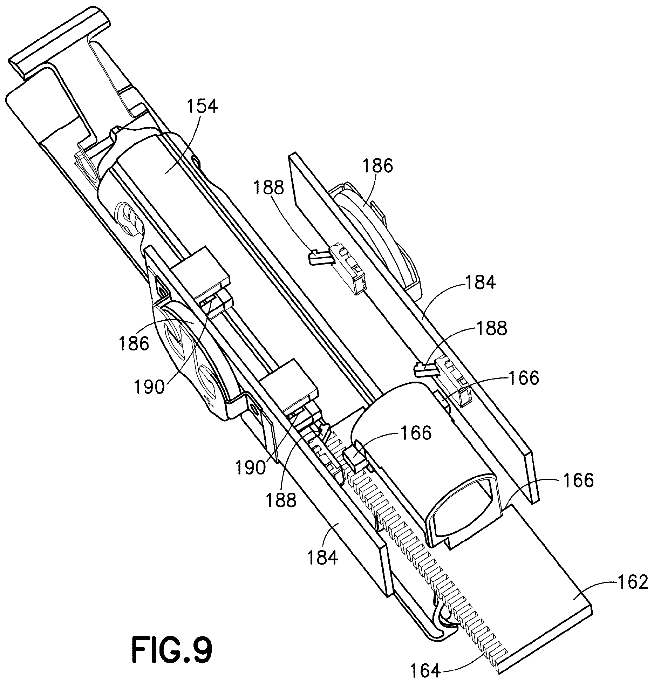

Turning now to FIGS. 9-10, shown are plunger body 150 and digital module 180 (with housing 182 not shown). In embodiments, plunger body 150 includes a rack 162. In non-limiting embodiments, rack 162 may include one or more teeth 164. Rack 162 may be arranged on plunger body 150 in a manner that allows for one or more teeth 164 to interact with one or more optical sensors 190. In non-limiting embodiments, rack 162 and/or plunger body 150 may also include one or more flanges 166. Plunger body 150 and/or rack 162 may be arranged in a manner that allows for one or more flanges 166 to interact with one or more mechanical switches 188. In the non-limiting embodiment of FIGS. 8-11B, optical sensors are slotted optical sensors, arranged such that one or more teeth 164 of rack 162 pass between two opposed sensors 190.

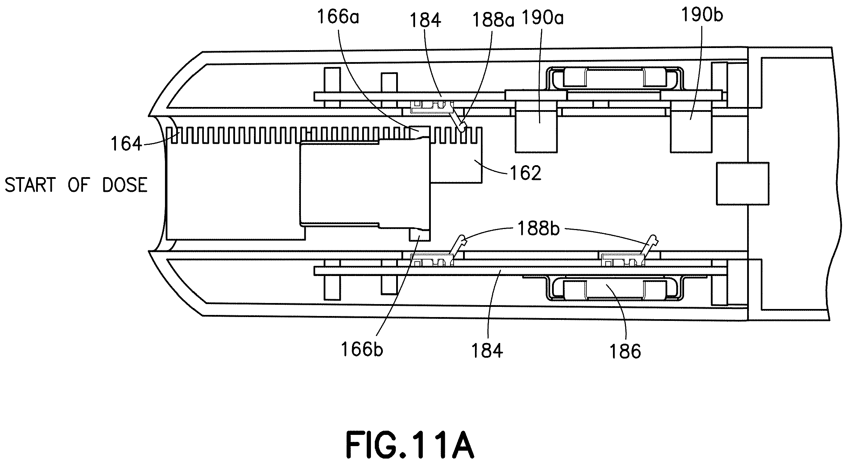

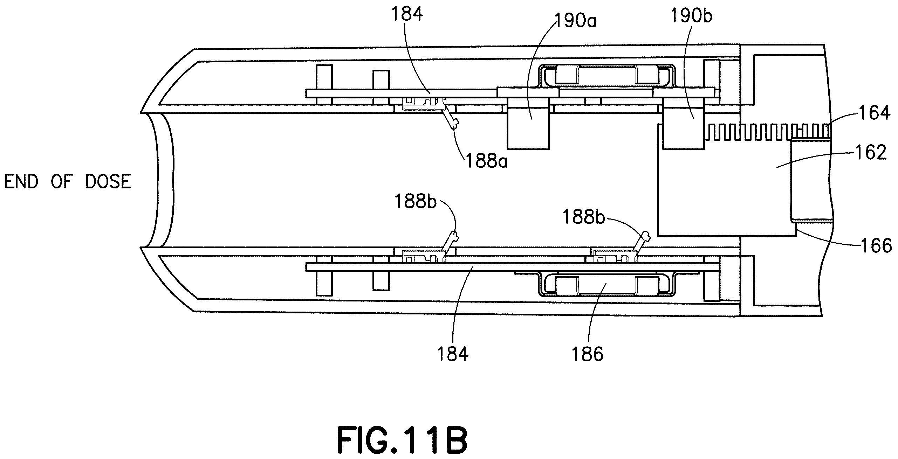

Referring now to FIGS. 11A-11B, shown are images of drug delivery device 100 in a pre-use (FIG. 11A) and post-use (FIG. 11B) position, with respect to the digital module 180. As shown in FIG. 11A, prior to actuation of the drive assembly, rack 162 is in a proximal position, and mechanical switches 188a, b have not been actuated. After drive assembly is actuated, plunger body 150, with rack 162, begins moving distally. One or more flanges 166a, b, c contact one or more mechanical switches 188a, b, causing the digital module to awaken and begin recording data (as will be described in greater detail below). As will be described in greater detail below (with reference to FIGS. 12-15), switches may be arranged, and processors included on circuit boards 184 may be programmed, to determine just the beginning and end of drug delivery, and/or may be arranged and programmed to continuously record data throughout drug delivery. In non-limiting embodiments, both arrangements may be included in the same digital module, for example as shown in FIGS. 7A-7B. In the illustrated embodiment of FIGS. 11A-11B, one or more teeth 164 on rack 162 interact with one or more optical sensors 190a, b, in the illustrated embodiment slotted optical sensors, and data may thus be continuously recorded during the drug delivery procedure. In the non-limiting embodiment of FIGS. 11A-11B, mechanical switch 188c falls off the end of the rack 162 after the proximal end thereof passes by, indicating the end of drug delivery.

With reference to FIGS. 12-13D, shown are non-limiting embodiments of arrangements of switches 188, 190, and interactions between the arranged switches 188,190 and teeth 164 and/or flanges 166 of a plunger body. As noted above, the arrangement of switches 188, 190 can be modified to provide an indication of a beginning of dose and end of dose only, a beginning of dose, end of dose, and continuous monitoring of the dose as it is delivered, or any combination thereof. In the non-limiting embodiment of FIG. 12, mechanical switch 188 is actuated by flange 166 when drug delivery begins. Which can wake a processor provided on one or more of the circuit boards. Thereafter, optical sensor 190 detects the passage of tooth 164 as rack, 162, with plunger body 150, moves distally to expel drug from the syringe 116. Thus, the embodiment of FIG. 12 can provide an indication of beginning of dose delivery, as well as continuous monitoring of the dose, as each tooth 164 passes, and is sensed by, optical switch 190.

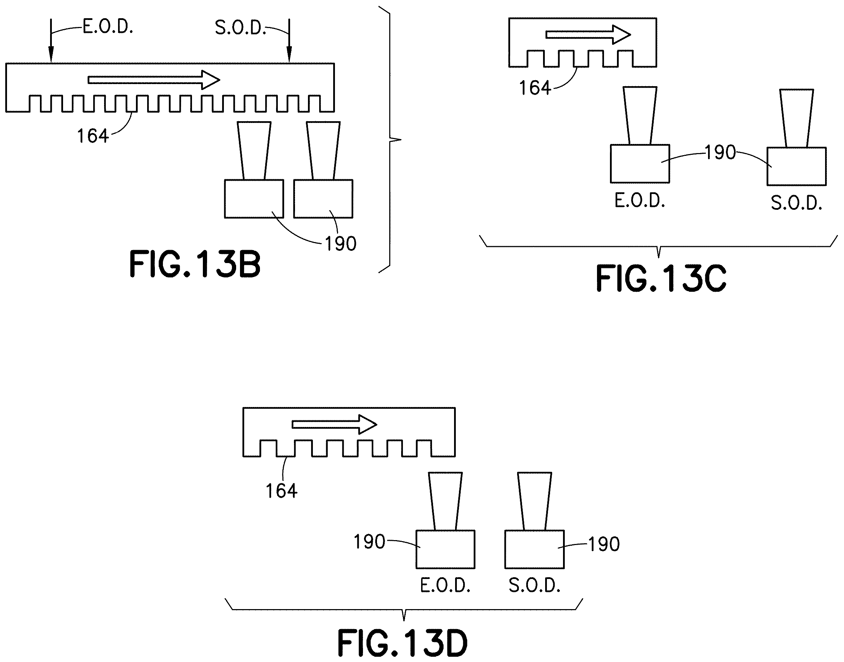

Turning now to FIGS. 13A-13D, shown are further non-limiting embodiments of arrangements of switches 188, 190, and interactions between the arranged switches 188,190 and teeth 164 and/or flanges 166 of a plunger body. FIG. 13A shows a non-limiting embodiment where only a single optical switch 190 is used to provide continuous monitoring throughout dose delivery. FIG. 13B shows use of two optical switches 190, arranged such that the switches are 90° out of phase, to provide continuous monitoring throughout dose delivery. This arrangement may provide increased resolution, and may also be suitable for determining direction of travel of plunger body 150. FIG. 13C shows use of two optical switches 190 with minimal overlap to provide continuous monitoring throughout dose delivery. The proximal-most switch 190 (S.O.D.) monitors the first half of dose delivery, and the distal-most switch 190 (E.O.D.) monitors the second half of dose delivery. FIG. 13D shows use of two optical switches that overlap to provide continuous monitoring throughout dose delivery. The proximal-most switch 190 (S.O.D.) monitors dose delivery, and near the end of dose delivery, distal-most switch 190 (E.O.D.) provides improved resolution, as plunger body 150 slows (due to drive member providing less force).

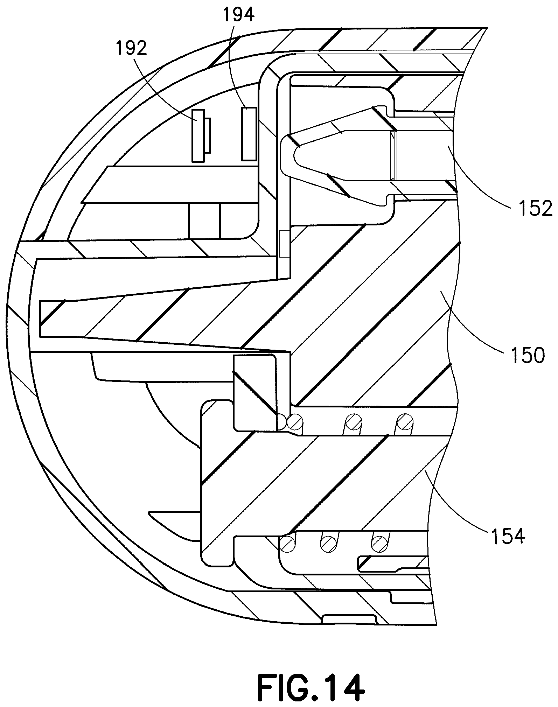

Turning to FIG. 14, shown is a non-limiting embodiment in which circuit board 184 includes, rather than (or in addition to) switches 188, 190, a camera 192. Camera 192, which may be a Time of Flight (ToF) camera, may interact with a reflective element 194, such as a mirror, arranged on plunger body 150, to detect movement of plunger body 150 during dose delivery. While a camera 192 is exemplified, those of skill will appreciate that other optical distance measuring systems, such as light detection and ranging (LIDAR), may be used to determine distance between a fixed point and plunger body 150 during dose delivery, and that time data can be generated and transmitted therefrom as described below.

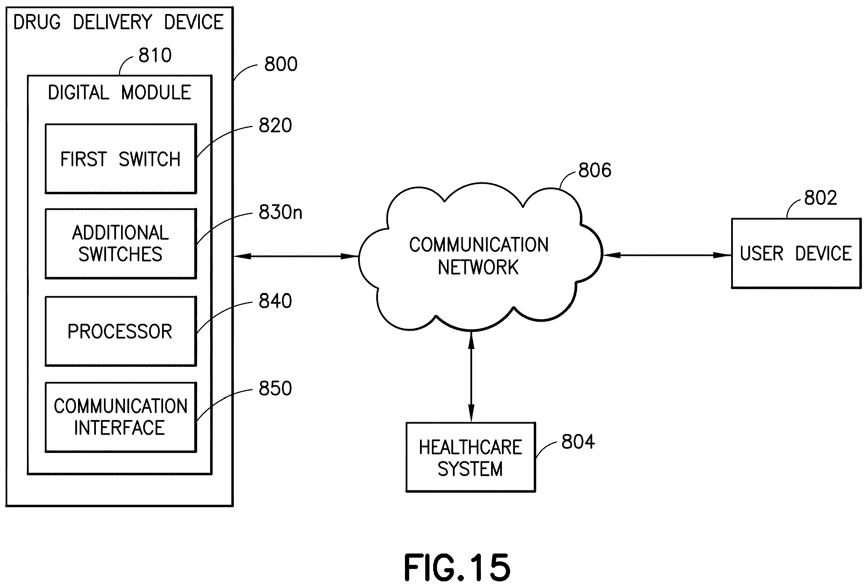

Referring now to FIG. 15, shown is a diagram of an example environment in which devices, systems, and/or methods, described herein, may be implemented. As shown in FIG. 15, the environment can include drug delivery device 800, user device 802, healthcare system 804, and/or communication network 806. Drug delivery device 800, user device 802, and healthcare system 804 may interconnect (e.g., establish a connection to communicate) via wired connections, wireless connections, or a combination of wired and wireless connections.

Drug delivery device 800, which can be an autoinjector as described herein, can include, as described above, digital module 810 including a first switch 820, optional additional switches 830n, processor 840, and communication interface 850. Drug delivery device 800 can be configured to communicate with a user device 802, such as a computing device as described herein.

Communication network 806 may include one or more wired and/or wireless networks. For example, communication network 806 may include a BLUETOOTH connection (e.g., between drug delivery device 800 and user device 802), a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a low-power wide area network (LPWAN), an ultra-wideband network (UWB), a metropolitan area network (MAN), a telephone network (e.g., the public switched telephone network (PSTN) and/or the like), a private network, an ad hoc network, an intranet, the Internet, a fiber optic-based network, a cloud computing network, and/or the like, and/or a combination of some or all of these or other types of networks.

User device 802 can be a computing device as described herein, in some non-limiting embodiments, a smartphone, smartwatch, tablet, laptop computer, desktop computer, or other computing device. User device 802 can be programmed or configured to communicate, for example through communication network 806, with a healthcare system 804, for example through an application, such as a mobile application, executable on user device 802.

Healthcare system 804 may include a server, a group of servers, and/or other like devices. More than one healthcare system 804 can be provided, for example, a system associated with a device manufacturer, a system associated with a pharmaceutical manufacturer, a system associated with a healthcare provider, a system associated with clinical research, a system associated with a government agency, and/or a system associated with a study sponsor, for example a sponsor of a clinical trial.

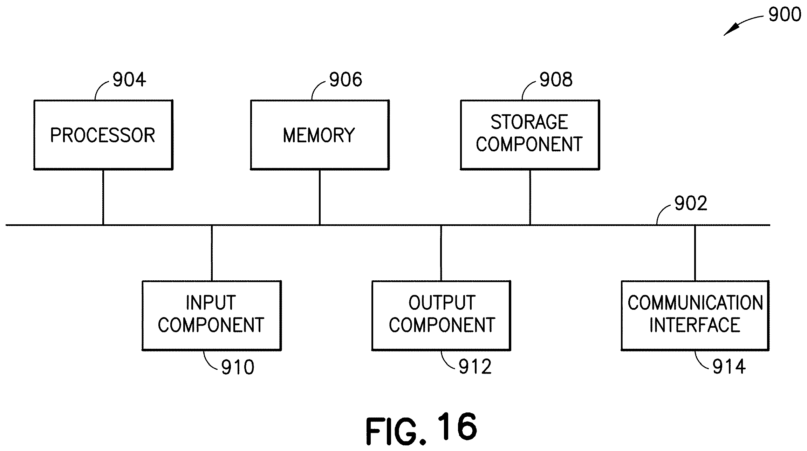

Referring now to FIG. 16, shown is a diagram of example components of an exemplary computing device 900 that can be used in the systems, devices, and methods described herein. Such a computing device 900 may correspond to component within a connection module of a drug delivery device as described herein, a user device as described herein, and/or a healthcare system as described herein. As shown in FIG. 9, a computing device 900 may include bus 902, processor 904, memory 906, storage component 908, input component 910, output component 912, and/or communication interface 914.

Bus 902 may include a component that permits communication among the components of computing device 900. In some non-limiting embodiments, processor 904 may be implemented in hardware, software, or a combination of hardware and software. For example, processor 904 may include a processor (e.g., a central processing unit (CPU), a graphics processing unit (GPU), an accelerated processing unit (APU), and/or the like), a microprocessor, a digital signal processor (DSP), and/or any processing component (e.g., a field-programmable gate array (FPGA), an application-specific integrated circuit (ASIC), and/or the like) that can be programmed to perform a function. Memory 906 may include random access memory (RAM), read-only memory (ROM), and/or another type of dynamic or static storage memory (e.g., flash memory, magnetic memory, optical memory, and/or the like) that stores information and/or instructions for use by processor 904.

Storage component 908 may store information and/or software related to the operation and use of computing device 900. For example, storage component 908 may include a hard disk (e.g., a magnetic disk, an optical disk, a magneto-optic disk, a solid state disk, and/or the like), a compact disc (CD), a digital versatile disc (DVD), a floppy disk, a cartridge, a magnetic tape, and/or another type of computer-readable medium, along with a corresponding drive.

Input component 910 may include a component that permits computing device 900 to receive information, such as via user input (e.g., a touch screen display, a keyboard, a keypad, a mouse, a button, a switch, a microphone, and/or the like). Additionally or alternatively, input component 910 may include a sensor for sensing information (e.g., a global positioning system (GPS) component, an accelerometer, a gyroscope, an actuator, a microphone, an environmental sensor (e.g., temperature, humidity, and the like), and/or the like). Output component 912 may include a component that provides output information from a computing device (e.g., a display, a speaker, one or more light-emitting diodes (LEDs), a tactile indicator, and/or the like).

Communication interface 914 may include a transceiver-like component (e.g., a transceiver, a separate receiver, and transmitter, etc.) that enables the device to communicate with other devices, such as via a wired connection, a wireless connection, or a combination of wired and wireless connections. Communication interface 914 may permit computing device 900 to transmit and/or receive information from another device. For example, communication interface 914 may include an Ethernet interface, an optical interface, a coaxial interface, an infrared interface, a radio frequency (RF) interface, a universal serial bus (USB) interface, a Wi-Fi^{®} interface, a cellular network interface, BLUETOOTH interface, and/or the like. In non-limiting embodiments, communication interface 914 does not operate through near field communication. Suitable communication protocols and methods for securing communications between communication interface 914 and a communication interface of another device, such as a computing device (e.g., desktop computer, laptop computer, smartphone, smart watch, PDA, tablet, etc.,) can include encryption, e.g., using a secure socket layer (SSL) (e.g., by using public/private key pairs as are known in the art). Additional security protocols are disclosed in, for example, U.S. Patent Nos. 9,445,264 and 9,463,325, the contents of which are to be referred to. In non-limiting embodiments described herein, a communication interface 914 is provided on the plunger rod. In non-limiting embodiments, communication interface 914 is configured to transmit data, but is not configured to receive data transmitted by a computing device, for example a user's smartphone.

A computing device may perform one or more processes described herein. A computing device may perform these processes based on processor 904 executing software instructions stored by a computer-readable medium, such as memory 906 and/or storage component 908, and/or being instructed by a separate computing device. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 906 and/or storage component 908 from another computer-readable medium or from another device via communication interface 914. When executed, software instructions stored in memory 906 and/or storage component 908 may cause processor 904 to perform one or more processes described herein. Additionally or alternatively, hardwired circuitry may be used in place of or in combination with software instructions to perform one or more processes described herein. Thus, embodiments described herein are not limited to any specific combination of hardware circuitry and software.

With reference to FIG. 15, in non-limiting embodiments, drug delivery device 800 includes a digital module 810 as described above, including a first switch 820 and additional switch 830n in communication with processor 840. Processor 840 is in communication with communication interface 850. The digital module 810, through communication interface 850, may thus be in communication with a user's computing device 802, such as a smartphone, having its own associated communication interface and processor, as well as memory, storage component, bus, input component, and/or output component. In non-limiting embodiments, communication interface 850 may also be in communication with healthcare system 804.

In non-limiting embodiments, digital module 810 is in one-way communication with a user's computing device 802 and/or healthcare system 804, e.g., the digital module 810 can only transmit data to the user device 802 and/or healthcare system 804, and cannot receive data from the user device 802 and/or healthcare system 804. In non-limiting embodiments, a processor associated with user device 802 can be programmed or configured, for example based on a mobile application stored in memory and/or storage component of user device 802, to place communication interface associated with user device 802 in a sleep mode, until communication interface 850 transmits data from digital module 810.

In non-limiting embodiments, mechanical switch(es) 188 may be used to determine assembly time of drug delivery device 100, for example of switch(es) 188 are actuated during the assembly process, and time data from assembly may be recorded and processed as described below.

In non-limiting embodiments, upon actuation of drug delivery device 800, first switch 820 is activated. As first switch 820 is in communication with processor 840, processor 840 receives a first signal from first switch 820, and optionally generates first time data, and optionally transmits, with communication interface 850, first time data, optionally with one or more identifiers (e.g., a device identifier and/or an identifier associated with the communication interface 850), to user device 802 and/or healthcare system 804. In non-limiting embodiments, additional data, such as data relating to power level of the power source(s), a counter value, and/or other data relating to the medicament(s) included (e.g., expiration date, lot number, manufacturer, etc.) and/or the functioning of drug delivery device 800 (e.g., position as determined by GPS, device orientation, or the like), is transmitted as well. In non-limiting embodiments, first time data and/or one or more identifiers are encrypted before being transmitted to user device 802. In non-limiting embodiments, processor accepts the data transmitted by communication interface 850 of drug delivery device 800 only if certain conditions are met. In non-limiting embodiments, the one or more conditions can include that any message received from drug delivery device 800 includes one or more of an identifier associated with the communication interface 850, an identifier associated with the drug delivery device 800, and/or a first identifier (associated, for example, with first signal), associated with activation of the first switch 820. In non-limiting embodiments, the message received from drug delivery device upon activation of first switch 820 is accepted by user device 802 and/or healthcare system 804 if, following a check, the message is the first transmitted message including the first identifier, indicating that the message has not previously been transmitted by drug delivery device 800. In non-limiting embodiments, the message is accepted by user device 802 and/or healthcare system 804 if, following a check, the combination of the first identifier and an identifier associated with the drug delivery device 800 and/or communication interface 850 has not previously been transmitted to user device 802 and/or healthcare system 804.

In non-limiting embodiments, user device 802 can, with a processor (e.g, processor 904), generate a first timestamp from the first time data, and may transmit the first timestamp (optionally with one or more identifiers associated with drug delivery device 800 as described herein) to healthcare system 804. In non-limiting embodiments, when first timestamp is transmitted to healthcare system 804, user device 802 terminates any connection with digital module 810. In non-limiting embodiments, for example those where continuous monitoring is provided, drug delivery device 800 continuously collects, and optionally continuously transmits, data during a drug delivery process. In such embodiments, the above may be repeated, e.g., second, third, etc. timestamps may be generated based on second, third, etc. time data, and such data and/or timestamps may be transmitted, optionally with any other data described herein, to user device 802 and/or healthcare system 804.

In non-limiting embodiments, upon reaching the end of the drug delivery process, first switch 820, or an additional switch (optical or mechanical) 830n, is actuated. As first switch 820 or additional switch 830n is in communication with processor 840, processor 840 receives a signal from first switch 820 or additional switch 830n as described above, and final time data is generated. Processor then transmits, with communication interface 850, final time data, optionally with one or more identifiers (e.g., a device identifier and/or an identifier associated with the communication interface 850), to user device 802. In non-limiting embodiments, final time data and/or one or more identifiers are encrypted before being transmitted to user device 802 and/or healthcare system 804. As discussed above, in non-limiting embodiments, user device 802 and/or healthcare system 804 accepts the data transmitted by drug delivery device 800 only if certain conditions are met. In non-limiting embodiments, the one or more conditions can include that any message received from drug delivery device 800 includes one or more of an identifier associated with the communication interface 850, an identifier associated with the drug delivery device 800, and/or a second identifier associated with activation of additional switch 830n. In non-limiting embodiments, the message received from drug delivery device upon activation of additional switch 830n is accepted by user device 802 and/or healthcare system 804 (and, in non-limiting embodiments, communication is reestablished between drug delivery device 800 and user device 802 and/or healthcare system 804) because such message is the first transmitted message including the second identifier, indicating that the message has not previously been transmitted by drug delivery device 800. In non-limiting embodiments, user device 802 and/or healthcare system 804 can, with a processor (e.g, processor 904), generate a final timestamp from the final time data, and, in the case of user device 802, may transmit the final timestamp (optionally with one or more identifiers associated with drug delivery device 800 as described herein) to healthcare system 804. In non-limiting embodiments, when final timestamp is transmitted to healthcare system 804, user device 802 terminates any connection with digital module 810.

In non-limiting embodiments, user device 802 transmits final timestamp and/or duration data to healthcare system 804 (optionally with one or more identifiers associated with drug delivery device 810 as described herein). In non-limiting embodiments, healthcare system can verify the timestamp data and/or duration data, for example by comparing the timestamp data, duration data, plunger rod travel data (e.g, speed and/or acceleration), and/or combinations thereof to predetermined ranges/values for each. In non-limiting embodiments, data falling outside of the predetermined ranges can be marked for follow up, or a message can be sent to user device 802 noting the issue.

In non-limiting embodiments, healthcare system 804 may be in communication with one or more additional healthcare systems 804. For example, one healthcare system may be maintained by the device manufacturer, and may be in communication with a healthcare system maintained by a study sponsor, pharmaceutical manufacturer, or the like. In non-limiting embodiments, timestamp (first timestamp, second timestamp, and/or final timestamp) and/or duration data, together with one or more identifiers (e.g., a device identifier and/or an identifier associated with the communication interface 850), may be transmitted from device manufacturer system to study sponsor or pharmaceutical manufacturer system, where one or more databases may store data associating the one or more identifiers (e.g., a device identifier and/or an identifier associated with the communication interface 850) with a patient identifier, allowing for association of timestamp and/or duration data with the patient.

Time data that is generated may be used in a variety of manners, for example, to predict time until end of dose delivery, to determine or estimate total dispensed volume of medicament, to calculate continuous and/or average flow rate of medicament during dose delivery, removal of drug delivery device form the patient's skin (e.g., by detecting an increase in plunger speed), and determining back pressure during dose delivery. Drug delivery devices as described herein may be equipped with one or more indicators, such as audible, tactile, and/or visual indicators, that may, in combination with the data that is collected and generated, provide feedback to the patient, for example to increase dwell time so that a full dose is known to have been delivered.

In addition to switches as described herein, drug delivery devices according to the present disclosure may also include one or more environment (e.g., temperature and/or humidity) sensors, GPS units, and/or gyroscopes (e.g., for determining injection orientation).

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

## Claims

1. A digital module for an autoinjector, comprising:
a housing (182) having a distal end and a proximal end and configured to releasably mate with an autoinjector (100) having a drive assembly; and
a first circuit board (184) received within the housing (182), the circuit board (184) having a proximal end and a distal end and comprising:
at least one switch (188, 190);
at least one processor (840) in communication with the at least one switch (188, 190);
at least one communication interface (850); and
at least one power source (186);
the digital module further comprises:
a second circuit board (184) comprising:
at least one switch (188, 190);
at least one processor (840) in communication with the at least one switch (188, 190);
at least one communication interface (850); and
at least one power source (186),
**characterized in that** circuit boards (184) are arranged in the housing (182) in a manner that provides a channel between the two circuit boards (184), wherein the first circuit board and the second circuit board are received within the housing on opposite sides of the channel, with one or more of the switches (188, 190) arranged facing the channel for allowing for components of the drive assembly of the drug delivery device (100) to be received in the channel, and to interact with the switches (188, 190), wherein the at least one switch (188, 190) of the first circuit board (184) comprises first and second mechanical switches (188) configured to engage a flange (166) of the drive assembly of the autoinjector (100), and wherein the at least one switch (188, 190) of the second circuit board (184) comprises first and second optical switches (190) configured to provide continuous monitoring of dose delivery of the autoinjector (100).

2. The digital module of claim 1, wherein at least one of the first circuit board (184) and the second circuit board (184) is a printed circuit board.

3. An autoinjector comprising:
an autoinjector housing (112, 142) having a proximal end and a distal end;
a syringe (116) comprising a barrel (117), a stopper (122), and a needle (120) arranged at a distal end of the syringe (116), at least a portion of the syringe (116) positioned within the housing (112, 142);
a drive assembly comprising a drive member (156) and a plunger body (152), the drive assembly configured to move the stopper (122) within the barrel (116) upon actuation of the drive assembly, at least a portion of the drive assembly positioned within the housing (112, 142);
a needle cover (124) having a pre-use position where the needle (120) is positioned within the needle cover (124), an actuation position where the needle cover (124) has been shifted proximally, thereby allowing the drive assembly to be actuated, and a post-use position where the needle (120) is positioned within the needle cover (124); and
a digital module (180) according to claim 1 or 2, comprising a module housing (182) having a distal end and a proximal end and configured to releasably mate with the autoinjector housing (112, 142); and
wherein the at least one switch (188, 190) of the first circuit board is activated by the plunger body (152) as the plunger body (152) moves distally during actuation of the drive assembly, and wherein activation of the at least one switch (188, 190) of the first circuit board indicates beginning of dose delivery and/or end of dose delivery.

4. The autoinjector of claim 3, wherein the plunger body (152) comprises a rack (162) having a plurality of teeth (164), and wherein the at least one switch (190)of the second circuit board detects movement of the plunger body (152) based on passage of one or more teeth (164).

5. The autoinjector of claim 4, wherein the first and second optical switches (190) of the second circuit board are arranged such that the switches are out of phase with each other relative to the plurality teeth (164) of the rack (162).

6. The autoinjector of claim 3, wherein the plunger body (152) further comprises at least a first flange (166a, 166b, 166c) configured to actuate the at least one switch (188), and wherein actuation of the at least one switch (188) by the flange indicates (166a, 166b, 166c) beginning of dose delivery.

7. The autoinjector of claim 6, wherein the plunger body (152) comprises at least a second flange (166a, 166b, 166c) configured to actuate the at least one switch (188) of the first circuit board, and wherein actuation of the at least one switch (188) of the first circuit board by the flange (166a, 166b, 166c) indicates end of dose delivery.

8. The autoinjector of claim 3, wherein the plunger body (152) comprises a first and second flange (166a, 166b), the first and second flange (166a, 166b, 166c) arranged on opposite sides of the plunger body (152), and a third flange (166, 166c).

## Patentansprüche

1. Digitalmodul für einen Autoinjektor, umfassend:
ein Gehäuse (182), das ein distales Ende und ein proximales Ende aufweist, und konfiguriert ist, um lösbar mit einem Autoinjektor (100) gepaart zu werden, der eine Antriebsanordnung aufweist; und
eine erste Leiterplatte (184), die innerhalb des Gehäuses (182) aufgenommen ist, wobei die Leiterplatte (184) ein proximales Ende und ein distales Ende aufweist und umfasst:
mindestens einen Schalter (188, 190);
mindestens einen Prozessor (840) in Kommunikation mit dem mindestens einen Schalter (188, 190);
mindestens eine Kommunikationsschnittstelle (850); und
mindestens eine Leistungsquelle (186);
wobei das Digitalmodul weiter umfasst:
eine zweite Leiterplatte (184), umfassend:
mindestens einen Schalter (188, 190);
mindestens einen Prozessor (840) in Kommunikation mit dem mindestens einen Schalter (188, 190);
mindestens eine Kommunikationsschnittstelle (850); und
mindestens eine Leistungsquelle (186),
**dadurch gekennzeichnet, dass** Leiterplatten (184) in dem Gehäuse (182) auf eine Weise angeordnet sind, die einen Kanal zwischen den beiden Leiterplatten (184) bereitstellen, wobei die erste Leiterplatte und die zweite Leiterplatte innerhalb des Gehäuses auf gegenüberliegenden Seiten des Kanals aufgenommen sind, wobei einer oder mehrere der Schalter (188, 190) dem Kanal zugewandt angeordnet sind, um zu ermöglichen, dass Komponenten der Antriebsanordnung der Arzneimittelabgabevorrichtung (100) im Kanal aufgenommen werden und mit den Schaltern (188, 190) zusammenwirken, wobei der mindestens eine Schalter (188, 190) der ersten Leiterplatte (184) erste und zweite mechanische Schalter (188) umfasst, die konfiguriert sind, um einen Flansch (166) der Antriebsanordnung des Autoinjektors (100) einzurasten, und wobei der mindestens eine Schalter (188, 190) der zweiten Leiterplatte (184) erste und zweite optische Schalter (190) umfasst, die konfiguriert sind, um eine kontinuierliche Überwachung der Dosisabgabe des Autoinjektors (100) bereitzustellen.

2. Digitalmodul nach Anspruch 1, wobei mindestens eine der ersten Leiterplatte (184) und der zweiten Leiterplatte (184) eine gedruckte Leiterplatte ist.

3. Autoinjektor, umfassend:
ein Gehäuse (112, 142), das ein proximales Ende und ein distales Ende aufweist;
eine Spritze (116), die einen Zylinder (117), einen Stopfen (122) und eine Nadel (120) umfasst, die an einem distalen Ende der Spritze (116) angeordnet sind, wobei mindestens ein Abschnitt der Spritze (116) innerhalb des Gehäuses (112, 142) positioniert ist;
eine Antriebsanordnung, die ein Antriebselement (156) und einen Kolbenkörper (152) umfasst, wobei die Antriebsanordnung konfiguriert ist, um den Stopfen (122) innerhalb des Zylinders (116) bei Betätigung der Antriebsanordnung zu bewegen, wobei mindestens ein Abschnitt der Antriebsanordnung innerhalb des Gehäuses (112, 142) positioniert ist;
eine Nadelabdeckung (124), die eine Vorverwendungsposition, in der die Nadel (120) innerhalb der Nadelabdeckung (124) positioniert ist, eine Betätigungsposition, in der die Nadelabdeckung (124) proximal verschoben worden ist, wodurch der Antriebsanordnung ermöglicht wird, betätigt zu werden, und eine Nachverwendungsposition aufweist, in der die Nadel (120) innerhalb der Nadelabdeckung (124) positioniert ist; und
ein Digitalmodul (180) nach Anspruch 1 oder 2, das ein Modulgehäuse (182) umfasst, das ein distales Ende und ein proximales Ende aufweist und konfiguriert ist, um lösbar mit dem Autoinjektorgehäuse (112, 142) gepaart zu werden; und
wobei der mindestens eine Schalter (188, 190) der ersten Leiterplatte durch den Kolbenkörper (152) aktiviert wird, wenn sich der Kolbenkörper (152) während einer Betätigung der Antriebsanordnung distal bewegt, und wobei Aktivieren des mindestens einen Schalters (188, 190) der ersten Leiterplatte ein Beginnen der Dosisabgabe und/oder Ende der Dosisabgabe angibt.

4. Autoinjektor nach Anspruch 3, wobei der Kolbenkörper (152) eine Zahnstange (162) umfasst, die eine Vielzahl von Zähnen (164) aufweist, und wobei der mindestens eine Schalter (190) der zweiten Leiterplatte eine Bewegung des Kolbenkörpers (152) basierend auf dem Durchgang eines oder mehrerer Zähne (164) erkennt.

5. Autoinjektor nach Anspruch 4, wobei der erste und zweite optische Schalter (190) der zweiten Leiterplatte angeordnet sind, sodass die Schalter zueinander in Bezug zur Vielzahl von Zähnen (164) der Zahnstange (162) aus der Phase sind.

6. Autoinjektor nach Anspruch 3, wobei der Kolbenkörper (152) weiter mindestens einen ersten Flansch (166a, 166b, 166c) umfasst, der konfiguriert ist, um den mindestens einen Schalter (188) zu betätigen, und wobei Betätigen des mindestens einen Schalters (188) durch den Flansch ein Beginnen der Dosisabgabe angibt (166a, 166b, 166c).

7. Autoinjektor nach Anspruch 6, wobei der Kolbenkörper (152) mindestens einen zweiten Flansch (166a, 166b, 166c) umfasst, der konfiguriert ist, um den mindestens einen Schalter (188) der ersten Leiterplatte zu betätigen, und wobei Betätigen des mindestens einen Schalters (188) der ersten Leiterplatte durch den Flansch (166a, 166b, 166c) ein Ende der Dosisabgabe angibt.

8. Autoinjektor nach Anspruch 3, wobei der Kolbenkörper (152) einen ersten und zweiten Flansch (166a, 166b), wobei der erste und zweite Flansch (166a, 166b, 166c) an gegenüberliegenden Seiten des Kolbenkörpers (152) angeordnet sind, und einen dritten Flansch (166, 166c) umfasst.

## Revendications

1. Module numérique pour un auto-injecteur, comprenant :
un boîtier (182) ayant une extrémité distale et une extrémité proximale et configuré pour s'accoupler de manière amovible avec un auto-injecteur (100) ayant un ensemble d'entraînement ; et
une première carte de circuit (184) reçue dans le boîtier (182), la carte de circuit (184) ayant une extrémité proximale et une extrémité distale et comprenant :
au moins un commutateur (188, 190) ;
au moins un processeur (840) en communication avec l'au moins un commutateur (188, 190) ;
au moins une interface de communication (850) ; et
au moins une source d'alimentation (186) ;
le module numérique comprend en outre :
une seconde carte de circuit (184) comprenant :
au moins un commutateur (188, 190) ;
au moins un processeur (840) en communication avec l'au moins un commutateur (188, 190) ;
au moins une interface de communication (850) ; et
au moins une source d'alimentation (186),
**caractérisé**
**en ce que** les cartes de circuit (184) sont agencées dans le boîtier (182) de manière à fournir un canal entre les deux cartes de circuit (184), où la première carte de circuit et la seconde carte de circuit sont reçues dans le boîtier sur des côtés opposés du canal, un ou plusieurs des commutateurs (188, 190) étant agencés de manière à faire face au canal afin de permettre à des composants de l'ensemble d'entraînement du dispositif d'administration de médicament (100) d'être reçus dans le canal et d'interagir avec les commutateurs (188, 190), où l'au moins un commutateur (188, 190) de la première carte de circuit (184) comprend des premier et second commutateurs mécaniques (188) configurés pour s'engager avec une bride (166) de l'ensemble d'entraînement de l'auto-injecteur (100), et où l'au moins un commutateur (188, 190) de la seconde carte de circuit (184) comprend des premier et second commutateurs optiques (190) configurés pour assurer une surveillance continue de l'administration de dose de l'auto-injecteur (100).

2. Module numérique selon la revendication 1, dans lequel au moins l'une de la première carte de circuit (184) et de la seconde carte de circuit (184) est une carte de circuit imprimé.

3. Auto-injecteur comprenant :
un boîtier d'auto-injecteur (112, 142) ayant une extrémité proximale et une extrémité distale ;
une seringue (116) comprenant un cylindre (117), un bouchon (122) et une aiguille (120) agencée au niveau d'une extrémité distale de la seringue (116), au moins une partie de la seringue (116) étant positionnée dans le boîtier (112, 142) ;
un ensemble d'entraînement comprenant un élément d'entraînement (156) et un corps de piston (152), l'ensemble d'entraînement étant configuré pour déplacer le bouchon (122) dans le cylindre (116) lors de l'actionnement de l'ensemble d'entraînement, au moins une partie de l'ensemble d'entraînement étant positionnée dans le boîtier (112, 142) ;
un couvercle d'aiguille (124) ayant une position de pré-utilisation où l'aiguille (120) est positionnée dans le couvercle d'aiguille (124), une position d'actionnement où le couvercle d'aiguille (124) a été déplacé de manière proximale, permettant ainsi l'actionnement de l'ensemble d'entraînement, et une position de post-utilisation où l'aiguille (120) est positionnée dans le couvercle d'aiguille (124) ; et
un module numérique (180) selon la revendication 1 ou 2, comprenant un boîtier de module (182) ayant une extrémité distale et une extrémité proximale et configuré pour s'accoupler de manière amovible avec le boîtier d'auto-injecteur (112, 142) ; et
dans lequel l'au moins un commutateur (188, 190) de la première carte de circuit est activé par le corps de piston (152) à mesure que le corps de piston (152) se déplace de manière distale lors de l'actionnement de l'ensemble d'entraînement, et dans lequel l'activation de l'au moins un commutateur (188, 190) de la première carte de circuit indique le début de l'administration de dose et/ou la fin de l'administration de dose.

4. Auto-injecteur selon la revendication 3, dans lequel le corps de piston (152) comprend une crémaillère (162) ayant une pluralité de dents (164), et dans lequel l'au moins un commutateur (190) de la seconde carte de circuit détecte le mouvement du corps de piston (152) sur la base du passage d'une ou de plusieurs dents (164).

5. Auto-injecteur selon la revendication 4, dans lequel les premier et second commutateurs optiques (190) de la seconde carte de circuit sont agencés de sorte que les commutateurs soient déphasés entre eux par rapport à la pluralité de dents (164) de la crémaillère (162).

6. Auto-injecteur selon la revendication 3, dans lequel le corps de piston (152) comprend en outre au moins une première bride (166a, 166b, 166c) configurée pour actionner l'au moins un commutateur (188), et dans lequel l'actionnement de l'au moins un commutateur (188) par la bride (166a, 166b, 166c) indique le début de l'administration de dose.

7. Auto-injecteur selon la revendication 6, dans lequel le corps de piston (152) comprend au moins une deuxième bride (166a, 166b, 166c) configurée pour actionner l'au moins un commutateur (188) de la première carte de circuit, et dans lequel l'actionnement de l'au moins un commutateur (188) de la première carte de circuit par la bride (166a, 166b, 166c) indique la fin de l'administration de dose.

8. Auto-injecteur selon la revendication 3, dans lequel le corps de piston (152) comprend des première et deuxième brides (166a, 166b), les première et deuxième brides (166a, 166b, 166c) étant agencées sur des côtés opposés du corps de piston (152), et une troisième bride (166, 166c).
